# EUROPEAN PATENT APPLICATION

(11) **EP 2 614 897 A2**
(43) Date of publication of application: **17.07.2013**
(21) Application number: 13151266.7
(22) Date of filing: 15.01.2013
(51) Int. Cl.: B06B 1/06, A61B 8/00

(54) **Ultrasonic probe and manufacturing method thereof**

(30) Priority: 16.01.2012 KR 20120004751
(71) Applicant: Samsung Medison Co., Ltd., Gangwon-do 250-870 (KR)
(72) Inventor: Min, Hae Kee, 109-1002 Ulsan (KR)
(74) Representative: Lorenz, Markus

(57) **Abstract**

An ultrasonic probe including a matching layer providing conductivity via an electrode formed on a surface contacting a piezoelectric material, and a manufacturing method thereof. The method of manufacturing an ultrasonic probe includes forming kerfs on a surface of the matching layer, forming an electrode on the surface of the matching layer at which the kerfs are formed, or on an opposite surface thereof, and mounting the surface provided with the electrode on the piezoelectric material.

## Description

### BACKGROUND

### 1. Field

Exemplary embodiments of the present disclosure embody an ultrasonic probe to generate an image of an internal state of an object using ultrasound.

### 2. Description of the Related Art

An ultrasonic diagnosis apparatus irradiates an ultrasonic signal toward a target region of the interior of a body of an object from a position adjacent the surface of the body of the object, and non-invasively acquires an image related to soft tissue tomograms or a blood stream using information from a reflected ultrasonic signal (an ultrasonic echo signal).

The ultrasonic diagnosis apparatus is relatively small and inexpensive, displays images in real time and is highly safe (e.g., no radiation exposure); as compared to other image diagnosis apparatuses, such as, for example, an X-ray diagnosis apparatus, an X-ray computerized tomography (CT) scanner, a magnetic resonance image (MRI), and a nuclear medicine diagnosis apparatus, and is thus widely used in many medical disciplines such as, for example, heart diagnosis, celiac diagnosis, urinary diagnosis, and obstetrical diagnosis.

An ultrasonic diagnosis apparatus includes an ultrasonic probe transmitting an ultrasonic signal to an object and receiving an ultrasonic echo signal reflected by the object to acquire an ultrasonic image of the object.

The ultrasonic probe includes a piezoelectric layer in which a piezoelectric material(s) vibrates thereby converting between an electrical signal and an acoustic signal, a matching layer for reducing an acoustic impedance difference between the piezoelectric layer and the object so as to maximize transmission of ultrasonic waves generated by the piezoelectric layer to the object, a lens for concentrating ultrasonic waves travelling in a forward direction of the piezoelectric layer on a predetermined point, and a backing layer for preventing ultrasonic waves from travelling in a backward direction of the piezoelectric layer to prevent image distortion.

### SUMMARY

Therefore, it is an object of the present disclosure to provide an ultrasonic probe including a matching layer providing conductivity by forming an electrode on a surface contacting a piezoelectric material and a manufacturing method thereof.

Additional aspects of the present disclosure will be set forth in part in the description which follows and, in part, will be obvious from the description, or may be learned by practice of the present disclosure.

In accordance with one aspect of the present disclosure, an ultrasonic probe includes a piezoelectric material and a matching layer disposed on a front surface of the piezoelectric material, wherein an electrode is formed over a surface of the matching layer facing the piezoelectric material.

The piezoelectric material may be arranged as a one-dimensional or two-dimensional array, and the matching layer and the piezoelectric material may be arranged in the same array.

Intervals between elements constituting an array of the matching layer may be respectively the same as intervals between elements constituting an array of the piezoelectric material.

The matching layer may include at least two layers.

In accordance with another aspect of the present disclosure, a method of manufacturing an ultrasonic probe includes forming kerfs at a surface of a matching layer, forming an electrode over the surface of the matching layer at which the kerfs are formed, or over an opposite surface of the matching layer, and mounting the surface of the matching layer provided with the electrode on the piezoelectric material.

The forming kerfs at a surface of a matching layer may be performed by forming kerfs at the surface of the matching layer to process the matching layer in a one-dimensional or two-dimensional array.

The piezoelectric material may be processed in a one-dimensional or two-dimensional array by forming kerfs having the same pattern as that of the kerfs formed at the surface of the matching layer.

The matching layer may include at least two layers.

The method may further include filling the kerfs with a material, and cutting the matching layer in a transverse direction to remove a portion of the matching layer having a surface opposite to a surface at which the kerfs are formed so as to expose the material filling the kerfs.

In accordance with another aspect of the present disclosure, an ultrasonic probe may include a piezoelectric layer, a matching layer disposed on the piezoelectric layer, and a first electrode arranged between the matching layer and the piezoelectric layer in a thickness direction.

The ultrasonic probe may further include a backing layer disposed on the piezoelectric layer, the piezoelectric layer may be arranged between the backing layer and the first electrode in the thickness direction.

The ultrasonic probe may further include a second electrode arranged between the backing layer and the piezoelectric layer.

The first electrode may be configured as a ground electrode and the second electrode may be configured as a signal electrode.

The piezoelectric layer and matching layer may include respective kerfs which are aligned in the thickness direction.

The matching layer may be arranged between two portions of the first electrode in a thickness direction.

The first electrode may extend over a side surface of at least one of the piezoelectric layer and matching layer.

The first electrode may extend over a side surface of the backing layer.

The matching layer may include kerfs on a surface thereof, the first electrode may extend into the kerfs.

The first electrode may extend into the kerfs of the matching layer.

The first electrode may be exposed to the kerfs of the piezoelectric layer and unexposed to the kerfs of the matching layer.

In addition, efficient ultrasound radiation may be conducted by disposing only a matching layer in an ultrasound-radiating direction without disposing a printed circuit board (PCB).

### BRIEF DESCRIPTION OF THE DRAWINGS

These and/or other aspects of the present disclosure will become apparent and more readily appreciated from the following description of exemplary embodiments, taken in conjunction with the accompanying drawings of which:
FIG.'S 1A-1F are diagrams describing a process of manufacturing an ultrasonic probe according to an exemplary embodiment of the present disclosure;
FIG.'S 2A-2C are diagrams illustrating a ground flexible printed circuit board (FPCB) connected to an ultrasonic probe according to an exemplary embodiment of the present disclosure;
FIG. 3 is a perspective view of FIG. 1D;
FIG.'S 4A-4D are diagrams describing a process of manufacturing an ultrasonic probe according to another exemplary embodiment of the present disclosure;
FIG. 5 is a perspective view of FIG. 4C;
FIG.'S 6A-6D are diagrams describing a process of manufacturing an ultrasonic probe according to another exemplary embodiment of the present disclosure; and
FIG. 7 is a perspective view of FIG. 6C.

### DETAILED DESCRIPTION

Reference will now be made in detail to exemplary embodiments of the present disclosure, examples of which are illustrated in the accompanying drawings, wherein like reference numerals refer to like elements throughout.

Hereinafter, an ultrasonic probe and a method of manufacturing the same will be described.

An ultrasonic probe according to an exemplary embodiment of the present disclosure includes a piezoelectric layer 20, a matching layer 10 provided on a front surface of the piezoelectric layer 20, and a backing layer 30 provided on a rear surface of the piezoelectric layer 20.

Voltage generation in a designated material in response to applied mechanical pressure and mechanical deformation in response to applied voltage are respectively referred to as a piezoelectric effect and an inverse piezoelectric effect, and a material exhibiting such effects is referred to as a piezoelectric material.

That is, a piezoelectric material converts electrical energy into mechanical vibration energy or converts mechanical vibration energy into electrical energy.

The ultrasonic probe according to the present disclosure includes the piezoelectric layer 20 composed of a piezoelectric material(s) that generates ultrasonic waves in response to an electrical signal applied thereto by converting the electrical signal into mechanical vibration.

The piezoelectric material(s) constituting the piezoelectric layer 20 may include PZMT single crystals formed of lead zirconate titanate (PZT) ceramics, a solid solution of lead magnesium niobate, and lead titanate or PZNT single crystals formed of a solid solution of lead zinc niobate and lead titanate.

The piezoelectric layer 20 may have a single-layered or multi-layered stack structure.

In general, impedance and voltage may be easily controlled in the piezoelectric layer 20 having a stack structure, so that excellent sensitivity, a relatively high energy conversion rate, and a relatively soft spectrum may be obtained.

In addition, electrodes capable of applying electrical signals may be formed over the front and rear surfaces of the piezoelectric layer 20. When the electrodes are formed over the front and rear surfaces of the piezoelectric layer 20, one of the electrodes may be a ground electrode and the other may be a signal electrode.

The matching layer 10 is provided on the front surface of the piezoelectric layer 20. The matching layer 10 reduces an acoustic impedance difference between the piezoelectric layer 20 and an object so as to match acoustic impedances of the piezoelectric layer 20 and the object. Thus, ultrasonic waves generated at the piezoelectric layer 20 can be effectively transmitted to the object.

For this, the acoustic impedance of the matching layer 10 may have a middle value between the acoustic impedances of the piezoelectric layer 20 and the object.

The matching layer 10 may be formed of a glass or resin material. In addition, a plurality of matching layers 10 may be formed, and the matching layers 10 may be formed of different materials in order to change the acoustic impedance gradually (e.g., stepwise) from the piezoelectric layer 20 to the object.

An electrode may be formed over a surface of the matching layer 10 which contacts the piezoelectric layer 20 according to the present disclosure, which arrangement will be described later with reference to the drawings.

The piezoelectric layer 20 and the matching layer 10 may be arranged in a two-dimensional matrix array by a dicing process or in a one-dimensional array.

Although not shown in the drawings, a protective layer may be formed on a front surface of the matching layer 10. The protective layer may be an RF shield capable of preventing leakage of a high-frequency component generated in the piezoelectric layer 20 to the outside and of blocking inflow of an external high-frequency signal.

Furthermore, the protective layer may be formed by coating a conductive material on a surface of a film having moisture resistance and chemical resistance and may be a C/S film capable of protecting internal parts from water and chemicals used in disinfection, and the like.

Although not shown in the drawings, a lens may be formed on a front surface of the protective layer. The lens may have a convex shape in an ultrasound-radiating direction so as to concentrate the ultrasonic waves on a predetermined point, but the lens may also have a concave shape if, for example, the speed of sound is faster than that in the human body.

The backing layer 30 is formed on the rear surface of the piezoelectric layer 20. The backing layer 30 absorbs and scatters ultrasonic waves generated at the piezoelectric layer 20 and travelling in the backward direction of the piezoelectric layer 20. Accordingly, image distortion may be prevented.

The backing layer 30 may be fabricated as a multi-layered structure in order to improve ultrasonic wave attenuation or blocking effects.

An electrode for applying an electrical signal to the piezoelectric layer 20 may be formed on a front surface of the backing layer 30 which contacts the piezoelectric layer 20, or formed in the backing layer 30.

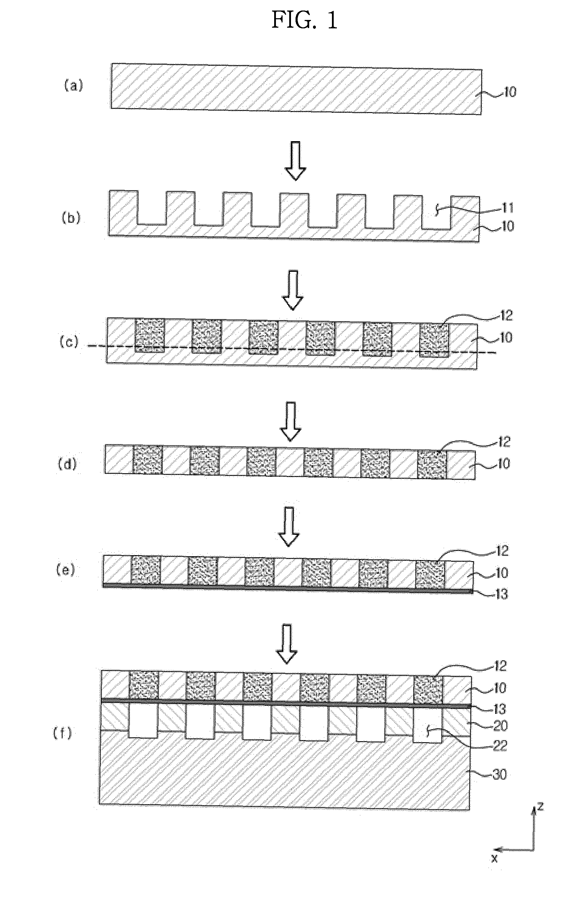

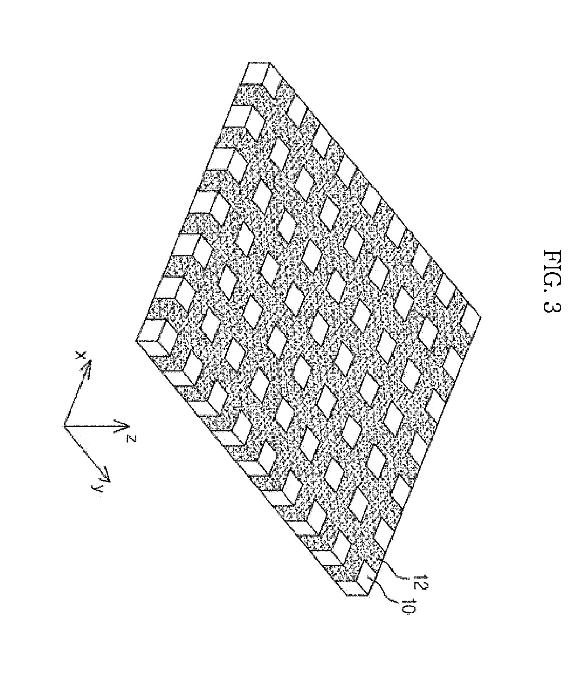

FIG.'S 1A-1F are diagrams for describing a process of manufacturing an ultrasonic probe according to an exemplary embodiment of the present disclosure. FIG.'S 2A-2C are diagrams illustrating a ground flexible printed circuit board (FPCB) connected to an ultrasonic probe according to an exemplary embodiment of the present disclosure. FIG. 3 is a perspective view of FIG. 1D.

As shown in FIG. 1, kerfs 11 are formed at one surface of a material used to form the matching layer 10 (FIG. 1B).

The kerfs 11 may be formed by a dicing process. With the formation of the kerfs 11, the matching layer 10 may be arranged as a one-dimensional or two-dimensional array. The width of each of the kerfs 11 formed at the matching layer 10 may be the same as that of kerfs 22 of the piezoelectric layer 20 which will be arranged in the same array as the matching layer 10. The kerfs 11 and 22 may be aligned in a thickness direction.

As shown in FIG. 1C, after the kerfs 11 are formed at the matching layer 10 (FIG. 1B), the kerfs 11 are filled with a material 12.

The kerfs 11 may be filled with any material 12 known for filling kerfs of ultrasonic probes.

As shown in FIG. 1D, after filling the kerfs 11, the matching layer 10is cut in a transverse direction along a dashed line shown in FIG. 1C to remove a portion of the matching layer 10 having a surface opposite to a surface at which the kerfs 11 are formed.

In the drawings, the transverse direction refers to a direction parallel to the xy plane. In this regard, the number of cuts or the cut surface(s) is not limited.

FIG. 3 is a perspective view of the matching layer 10 formed by cutting the matching layer 10 to remove a portion of the matching layer 10 having a surface opposite to a surface at which the kerfs 11 are formed. Referring to FIG. 3, the matching layer 10 is arranged in a two-dimensional matrix array with the material filling the kerfs 11 being included at outer boundaries of the matching layer 10. The two-dimensional matrix array is just one example of an array that can be used. If the piezoelectric layer 20 is arranged as a one-dimensional array, the matching layer 10 may also be arranged as a one-dimensional array.

Any known cutting methods and other methods such as, for example, grinding, may be used for the cutting process.

After cutting the matching layer 10 to remove a portion of the matching layer 10 having a surface opposite to a surface at which the kerfs 11 are formed as shown in FIG. 1D and FIG. 3, an electrode 13 is formed on at least one of the front and rear surfaces of the matching layer 10 (FIG. 1E).

FIG. 1E illustrates an exemplary embodiment in which the electrode 13 is formed over the rear surface of the matching layer 10. In addition to the rear surface, the electrode 13 may also extend over side surfaces and/or over the front surface of the matching layer 10. The electrode 13 may be formed by coating or depositing a conductive material on the front, side or rear surface of the matching layer 10. According to this exemplary embodiment, the electrode 13 is formed on the rear surface of the matching layer 10 by sputtering.

FIG. 1 shows a single-layered matching layer 10. However, the matching layer 10 may also have two or more layers.

The matching layer 10 having at least one surface provided with the electrode 13 as described above is mounted on the piezoelectric layer 20 (FIG. 1F).

The piezoelectric layer 20 provided with the backing layer 30 on the rear surface thereof may be arranged as a one-dimensional or two-dimensional array. As shown in FIG. 1F, the matching layer 10 and the piezoelectric layer 20 may be arranged in the same array, and the intervals of the kerfs 11 and 22 may be the same. Thus, elements of the matching layer 10 may correspond to those of the piezoelectric layer 20 in a one-to-one relationship.

An electrode may be formed on the front surface of the backing layer 30 contacting the piezoelectric layer 20 and may be used as a signal electrode to apply an electrical signal to elements constituting an array of the piezoelectric layer 20. Also, an electrode may be formed in the backing layer 30 to penetrate the backing layer 30 and may extend from the front surface to the rear surface of the backing layer 30, and may be used as a signal electrode to apply an electrical signal to elements constituting an array of the piezoelectric layer 20. Any signal electrode that applies an electrical signal to the piezoelectric layer 20 may be used, without limitation.

An ultrasonic probe according to an exemplary embodiment of the present disclosure may use the electrode 13 formed on the rear surface of the matching layer 10 as a ground electrode.

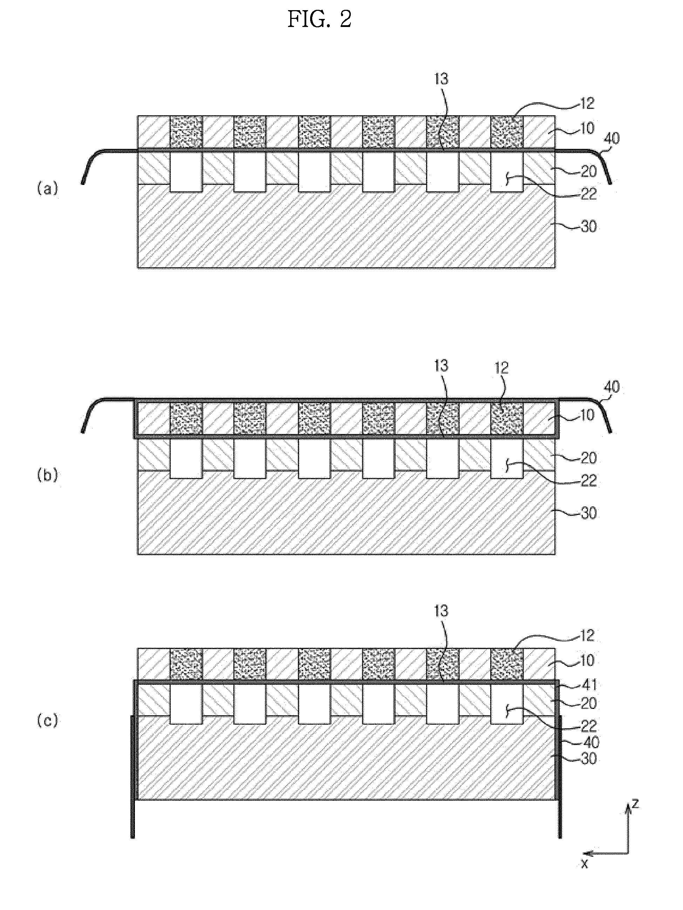

FIG. 2 illustrates various examples of grounding the electrode 13 formed on the matching layer 10. If the electrode 13 is formed on the rear surface of the matching layer 10 as shown in FIG. 1, the electrode 13 may be grounded by connecting the FPCB 40 to the electrode 13 formed on the rear surface of the matching layer 10 as shown in FIG. 2A.

In addition, if the electrode 13 is formed on the rear surface of the matching layer 10 while extending over side surfaces and the front surface of the matching layer 10, the electrode 13 may be grounded by connecting the FPCB 40 to the electrode 13 formed on the front surface of the matching layer 10 (FIG. 2B).

The electrode 13 may also be grounded by forming the electrode 13 on the rear surface of the matching layer 10 and including an electrode portion 41 of the electrode 13 which extends over side surfaces of the piezoelectric layer 20 and the backing layer 30, and connecting the electrode portion 41 formed on the side surfaces of the piezoelectric layer 20 and the backing layer 30 to the FPCB 40 (FIG. 2C).

The electrode 13 formed on the matching layer 10 may be grounded as described above, but various modified embodiments may also be made without limitation. The electrode 13 formed on the matching layer 10 may also be grounded, in addition to using the various exemplary methods described above, according to another embodiment of the present disclosure that will be described below.

As such, an electric signal may be applied to each of the elements constituting an array of the piezoelectric layer 20 through the electrode 13 formed on the matching layer 10 and an electrode formed on the backing layer 30 as described above.

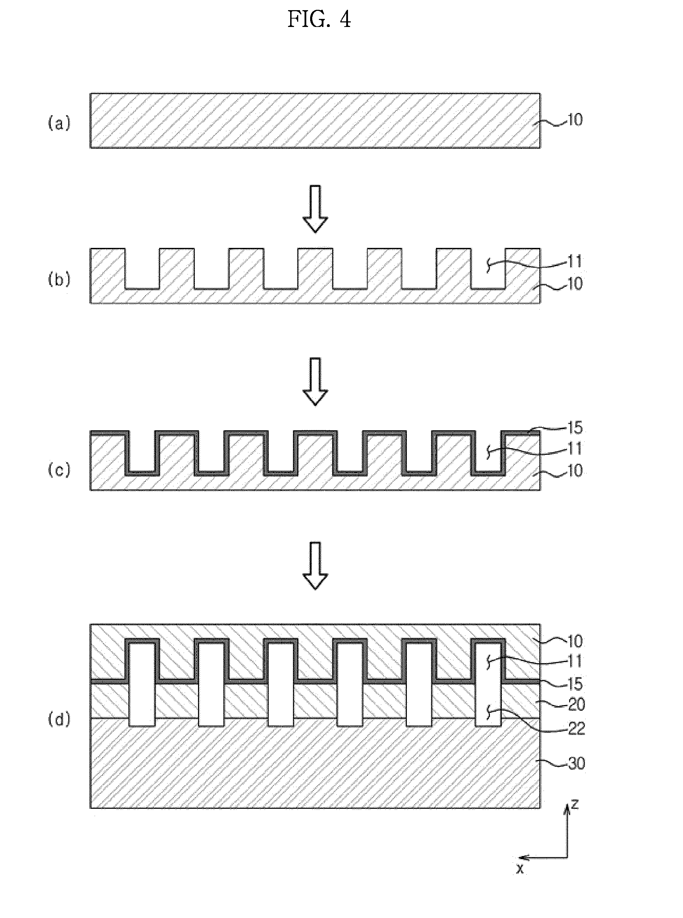

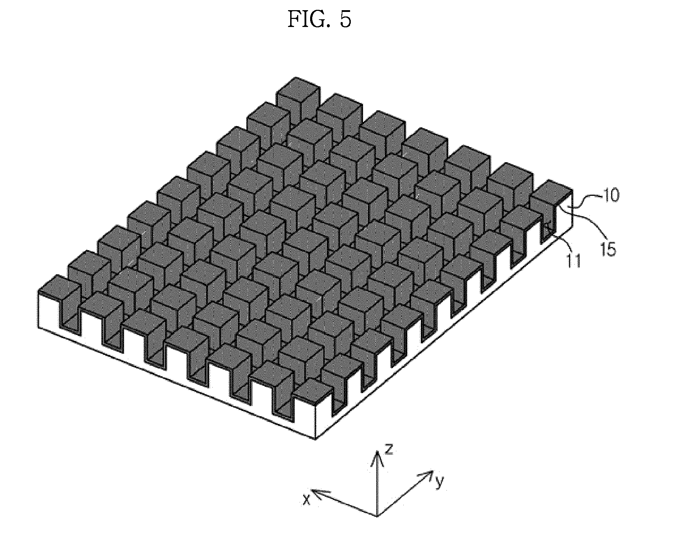

FIG.'S 4A-4D are diagrams describing a process of manufacturing an ultrasonic probe according to another exemplary embodiment of the present disclosure. FIG. 5 is a perspective view of FIG. 4C.

The process shown in FIG. 4 can be the same as that shown in FIG. 1 until the kerfs 11 are formed at the matching layer 10.

After formation of the kerfs 11 at the matching layer 10, an electrode 15 is formed on a surface of the matching layer 10 at which the kerfs 11 are formed, without filling the kerfs 11 with the material 12, so that the electrode 15 extends into the kerfs 11 (FIG. 4C).

The electrode 15 may be formed by coating or depositing a conductive material on the surface of the matching layer 10 at which the kerfs 11 are formed. The electrode 15 may be formed on the surface of the matching layer 10 by, for example, sputtering according to an exemplary embodiment. FIG. 5 is a perspective view of a structure in which the electrode 15 is formed on the surface of the matching layer 10 at which the kerfs 11 are formed. As shown in FIG. 5, the electrode 15 may be formed over the entire surface of the matching layer 10 arranged in a two-dimensional matrix array. The two-dimensional matrix array is just one example of an array that can be used. If the piezoelectric layer 20 is arranged as a one-dimensional array, the matching layer 10 may also be arranged as a one-dimensional array.

The matching layer 10 provided with the electrode 15 as described above is mounted on the front surface of the piezoelectric layer 20 such that the kerf-formed surface faces the piezoelectric layer 20 (FIG. 4D). As shown in FIG. 4D, the matching layer 10 and the piezoelectric layer 20 may be arranged in the same array and the intervals of the kerfs 11 and 22 may be the same. As a result, elements of the matching layer 10 may correspond to those of the piezoelectric layer 20 in a one-to-one relationship.

As described above with reference to the exemplary embodiment shown in FIG. 1, the ultrasonic probe according to the exemplary embodiment shown in FIG.'S 4-5 may use the electrode 15 of the matching layer 10 as a ground electrode. The signal electrode may be the same as that of the exemplary embodiment shown in FIG. 1, and thus the detailed description thereof is omitted.

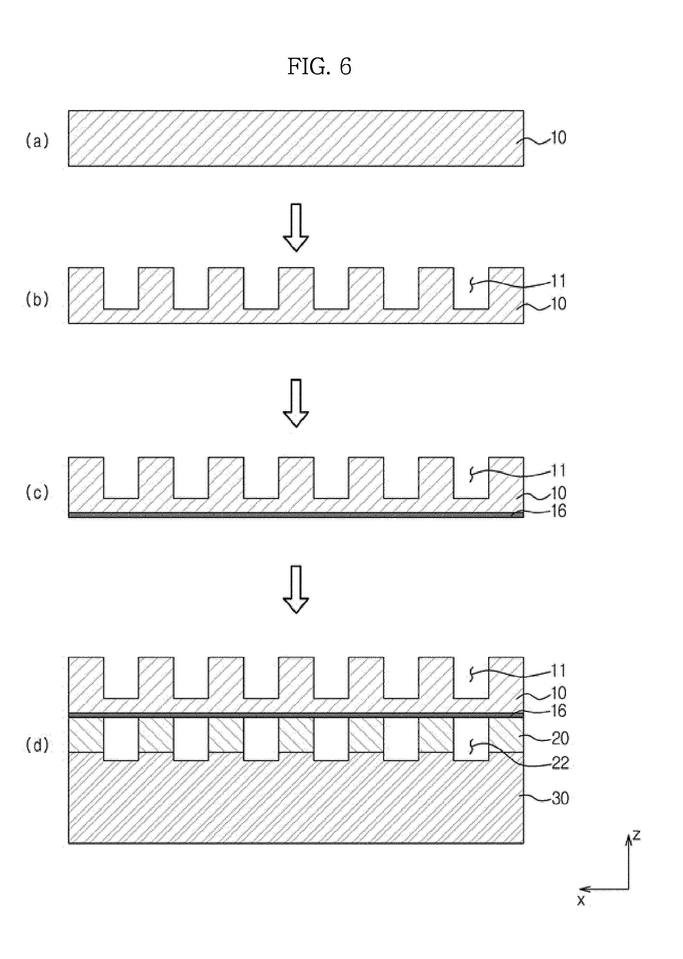

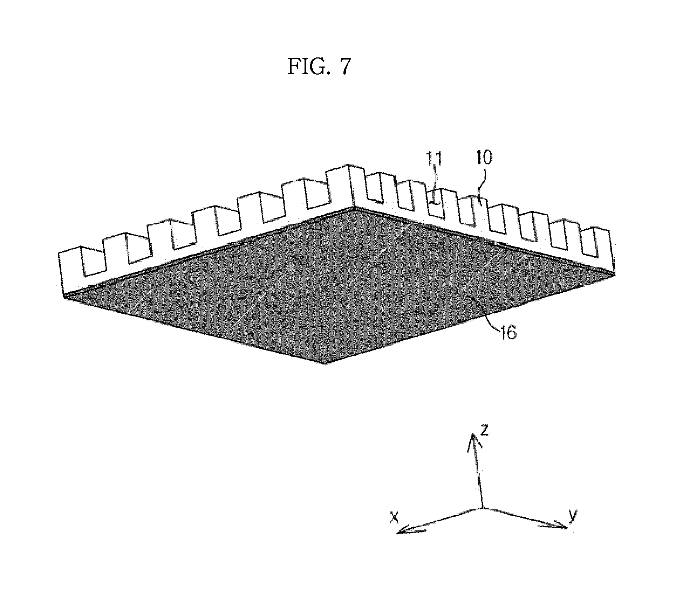

FIG.'S 6A-6D are diagrams describing a process of manufacturing an ultrasonic probe according to another exemplary embodiment of the present disclosure. FIG. 7 is a perspective view of FIG. 6C.

The process shown in FIG. 6 can be the same as that shown in FIG. 1 until the kerfs 11 are formed at the matching layer 10.

After formation of the kerfs 11 at the matching layer 10, an electrode 16 is formed on a surface of the matching layer 10 opposite to a surface at which the kerfs 11 are formed (FIG. 6C).

The electrode 16 may be formed by coating or depositing a conductive material over the surface of the matching layer 10 opposite to the surface at which the kerfs 11 are formed. According to an exemplary embodiment, the electrode 16 may be formed over the surface of the matching layer 10 by sputtering.

The matching layer 10 provided with the electrode 16 as described above is mounted on the front surface of the piezoelectric layer 20 (FIG. 6D). As shown in FIG. 6D, the matching layer 10 and the piezoelectric layer 20 may be arranged in the same array and the intervals of the kerfs 11 and 22 may be the same. As a result, elements of the matching layer 10 may correspond to those of the piezoelectric layer 20 in a one-to-one relationship.

As described above with reference to the exemplary embodiment shown in FIG. 1, the ultrasonic probe according to the exemplary embodiment shown in FIG.'S 6-7 may use the electrode 16 of the matching layer 10 as a ground electrode. The signal electrode may be the same as that of the exemplary embodiment shown in FIG. 1, and thus the detailed description thereof is omitted.

Although a few exemplary embodiments of the present disclosure have been shown and described, it would be appreciated by those skilled in the art that changes may be made in these embodiments without departing from the principles and spirit of the present disclosure, the scope of which is defined in the claims and their equivalents.

## Claims

1. An ultrasonic probe comprising:
a piezoelectric material; and
a matching layer disposed on a front surface of the piezoelectric material,
wherein an electrode is formed over a surface of the matching layer facing the piezoelectric material.

2. The ultrasonic probe according to claim 1, wherein the piezoelectric material is arranged as a one-dimensional or two-dimensional array, and
the matching layer and the piezoelectric material are arranged in the same array.

3. The ultrasonic probe according to claim 2, wherein intervals between elements constituting an array of the matching layer are respectively the same as intervals between elements constituting an array of the piezoelectric material.

4. The ultrasonic probe according to claim 1, wherein the matching layer comprises at least one layers.

5. A method of manufacturing an ultrasonic probe, the method comprising:
forming kerfs at a surface of a matching layer;
forming an electrode over the surface of the matching layer at which the kerfs are formed, or over an opposite surface of the matching layer; and
mounting the surface of the matching layer provided with the electrode on the piezoelectric material.

6. The method of claim 5, wherein the forming kerfs at a surface of a matching layer is performed by forming kerfs at the surface of the matching layer to process the matching layer in a one-dimensional or two-dimensional array.

7. The method of claim 5, wherein the piezoelectric material is processed in a one-dimensional or two-dimensional array by forming kerfs having the same pattern as that of the kerfs formed at the surface of the matching layer.

8. The method of claim 5, wherein the matching layer comprises at least one layers.

9. The method of claim 5, further comprising:
filling the kerfs with a material; and
cutting the matching layer in a transverse direction to remove a portion of the matching layer having a surface opposite to a surface at which the kerfs are formed so as to expose the material filling the kerfs.
